# EUROPEAN PATENT SPECIFICATION

(11) **EP 2 467 121 B1**
(45) Date of publication and mention of the grant of the patent: **31.08.2016**
(21) Application number: 10735299.9
(22) Date of filing: 28.07.2010
(51) Int. Cl.: A61K 8/02, A61K 8/92, A61K 8/04, A61Q 11/00

(54) **DELIVERY SYSTEM**
ABGABESYSTEM
SYSTÈME DE LIVRAISON

(30) Priority: 19.08.2009 EP 09168145
(43) Date of publication of application: 27.06.2012
(73) Proprietor: Unilever PLC, London EC4Y 0DY (GB); Unilever N.V., 3013 AL Rotterdam (NL)
(72) Inventor: GROVES, Brian, Joseph, Wirral Merseyside CH63 3JW (GB)
(74) Representative: Tansley, Sally Elizabeth
(86) International application number: PCT/EP2010/060951
(87) International publication number: WO 2011/020684

(56) References cited:
- EP-A1- 1 477 152
- EP-A1- 1 967 173
- WO-A1-93/00065
- WO-A1-2006/065239
- US-A- 5 665 687
- US-A1- 2006 264 346

## Description

The present invention relates to a friable delivery system for an active ingredient and to a method for manufacturing such a delivery system.

The present invention also concerns the use friable delivery systems in personal care compositions, particularly oral care compositions, and their use for delivering flavours, fragrances, and colourants.

WO 08/01586 (Groves) discloses oral care compositions comprising capsules having a core and a coating, the coating comprising was having a melting point of at least 70°C, an oil and an opacifier and the core comprising an oil, a pigment and a shear thinning structurant.

US 4,071,614 (Grimm) discloses dentifrices containing encapsulated flavourings.

US 6,060,084 (Sullivan et al) discloses a method for preparing core-shell containment systems comprising a shell material having a softening temperature above about 70°F.

GB 1,381,444 (Blendex-Werke R. Schneider & Co.) discloses toothpaste formulations comprising microcapsules having a transparent shell and encapsulating colouring matter.

US 3,914,405 (Shepherd and Gould) discloses toothpaste compositions comprising a flavouring agent encapsulated in a hydrophilic polymer.

WO 2005/123023 (Ribi) discloses a toothpaste composition comprising a material which is capable of changing colour on pH change.

In a first aspect the present invention, there is provided a delivery system comprising friable agglomerates, said friable agglomerates comprising oil, wax having a melting point of at least 50°C, and active ingredient, characterized in that the friable agglomerates have air pockets within them, wherein the air pockets comprise from 10 to 50% by volume of the agglomerates.

In a second aspect of the present invention, there is provided a method of delivering active ingredient comprising the incorporation of said active ingredient in friable agglomerates comprising oil and wax having a melting point of at least 50°C, the application of said friable agglomerates to the desired site of delivery, followed by the shear or pressure induced breakage of said friable granules, thereby releasing the active ingredient, characterized in that the friable agglomerates have air pockets within them.

In a third aspect of the present invention, there is provided a method of manufacture of a delivery system comprising the formation of a homogeneous mixture of oil, wax having a melting point of at least 50°C, and active ingredient, the addition of said homogeneous mixture to water with the incorporation of air bubbles into said homogeneous mixture, the homogeneous mixture and the water to which it is added both being at a temperature above the melting point of the wax, the cooling of the resulting aqueous dispersion to a temperature below the melting point of the wax, followed by the drying of the resulting waxy agglomerates.

The delivery system of the present invention is typically used to enable delayed release of active ingredient, the release being triggered by shear or pressure upon the agglomerates comprised within the delivery system. The breakage of the agglomerates, thereby releasing active ingredient, is aided by the air pockets within them.

The delivery system might alternatively be considered an encapsulation system, the active ingredient being held within the agglomerates until they are broken. It must be remembered, however, that the active ingredient is typically spread uniformly through the agglomerates, unlike the situation in many encapsulation systems.

The delivery system may be used in a range of compositions. It is particularly suitable for use in personal care compositions that are applied to the surface of the human body. During the use of such compositions, the shear or pressure required to break the agglomerates is typically provided by the hand rubbing or pressing the agglomerates against the skin.

The delivery system is especially suitable for use in oral care compositions, such as toothpastes. In toothpastes, the shear or pressure required to break the agglomerates is typically provided by a toothbrush, whether manual or electrical. The delivery system may be used to give delayed release of flavour on using toothpaste. In a particularly preferred use, the delivery system allows delayed release of colourant, in particular pigment, from toothpaste.

In certain preferred applications, the breakage of the agglomerates and consequential release of the active ingredient may be used as an indicator that the activity producing the shear or pressure has been carried out for the "desired" time. For example, when the delivery system is used in toothpaste and the activity producing the shear or pressure is tooth brushing, the release of the active ingredient may indicate to the consumer that he has brushed his teeth for the desired time. In this example, the active ingredient may be a colourant which changes the colour of the toothpaste-saliva mixture as it is released from the agglomerates. It is well established that most people do not brush their teeth for the minimum recommended time of two minutes. The present invention is capable of helping with this problem, by giving people a clear indication of when they have brushed for the recommended time.

The breakage of the agglomerates is most typically brought about by shear of the delivery agglomerates against a hard surface. When used in toothpaste, the agglomerates tend to be sheared against the teeth.

The agglomerates have an average yield stress of preferably from 1 x 10⁴ to 1 x 10⁷ Pa and more preferably from 1 x 10⁵ to 1 x 10⁶ Pa. The mechanical strength of the agglomerates may be varied by adjusting the volume of air pockets incorporated therein and/or by varying the wax:oil ratio. The average yield stress of the agglomerates is a measure of their friability.

The agglomerates may be present in an amount ranging from 0.01 to 50% by weight of the composition in which they are used. Preferably, they comprise from 0.5 to 10% by weight of the composition. When used in personal products, such an amount may provide the optimum sensory benefit during use and also the optimum release profile of the active ingredient when the agglomerates are broken.

The particle size of the agglomerates is preferably such that at least 90% by volume are in the range of from 300 to 800 microns. Measurement of the particle size distribution of the agglomerates may be made by laser light scattering techniques. It has been found that agglomerates smaller than 300 microns are not sufficiently broken down in preferred applications. It has also been found that agglomerates greater than 800 microns tend to have delivery problems in preferred applications. Use in toothpaste is a preferred application, particularly for delayed release of a colourant to the oral cavity during tooth brushing.

The agglomerates typically have a predominately spherical shape.

The oil is preferably obtained from an animal, vegetable or mineral source. Preferred sources are vegetable sources, such as fruits and plant seeds. Preferred oils are olive oil, borage oil, primrose oil, groundnut oil, canola oil, safflower oil, rice bran oil, peanut oil and other plant seed oils, including sunflower seed oil. The most preferred oil is sunflower seed oil, in particular the so-called high-oleate sunflower seed which presents a higher resistance to rancidity. The choice of oil is particularly important when the delivery system is used cosmetic products and is especially important when the delivery system is used in toothpaste.

The amount of oil in the agglomerates is preferably from 30 to 70%, more preferably from 40 to 60%, and most preferably from 43 to 53% by weight.

The wax is preferably one with a melting point of greater than 70°C, more preferably greater than 80°C. Preferred waxes include carnauba wax, Chinese insect wax, candililla wax, castor wax, Japan wax and synthetic waxes such as high molecular weight paraffin wax and microcrystalline wax. Particularly preferred waxes are castor wax 80 (melting point: 80°C) and carnauba wax (melting point: 80-85°C), with carnauba wax being most preferred.

Selecting the correct wax can give surprising benefits when the delivery system is used in certain compositions. For example, it has been found that carnauba wax gives agglomerates with surprisingly good stability in compositions also comprising anionic surfactants, such sodium laurylsulphate. When glycerol monostearate is used as the wax in agglomerates, the agglomerates are not suitable for use in compositions comprising anionic surfactants, the surfactant tending to destabilize such agglomerates.

The amount of wax in the agglomerates is preferably from 20 to 60%, more preferably from 25% to 50%, and most preferably from 30 to 40% by weight. The wax serves to structure the agglomerates, holding them together.

The preferred weight ratio between the wax and the oil is from 20:80 to 80:20, more preferably from 30:70 to 50:50. When the wax used is carnauba wax and the oil used in sunflower seed oil, the preferred ratio of wax to oil is preferably about 40:60. The ratio between the wax and oil affects the friability of the agglomerates and is important for giving the agglomerates the best resistance to fracture during processing and also the best active ingredient release profile.

The present invention allows the active ingredient to have a delayed release profile. This can give particular benefits when the active ingredient is a flavour or fragrance.

The present invention may also be used to protect the active ingredient from the environment of a composition in which it is placed. Hence, in some embodiments the active ingredient is a component having incompatibility with the composition in which its use is desired.

In certain embodiments, the active ingredient may be a colourant, releasing colour when the agglomerates are broken by shear or pressure. This can serve as a visible signal to the consumer that the activity producing the shear or pressure has been carried out for the "desired" time.

When the active ingredient is a colourant, it is preferred that it is a pigment. Pigments are typically insoluble in water and insoluble in the oil-wax mixture comprised in the agglomerates.

Preferred pigments are blue, red, green, yellow, purple or orange. The most preferred are blue. Especially preferred are those selected from CI 14720, CI 15985, CI 16035, CI 16255, CI 19140, CI 42051, CI 42053, CI 42090, CI 47005, CI 73360, CI 74160, CI 74260, CI 75470, CI 75810, CI 77007, CI 77491 and CI 77492. These pigments provide the optimum visual signal that tooth brushing has been carried out for the required time, when the agglomerates are used for this purpose in toothpaste.

The amount of active ingredient in the agglomerates is preferably from 0.1 to 30%, more preferably 1% to 25%, and most preferably from 5 to 20% by weight.

When the active ingredient is a pigment, it is preferred that the amount of pigment in the total composition is at most 0.3% and more preferably at most 0.2% by weight.

The active ingredient is typically spread uniformly throughout the agglomerates. It is preferred that the active ingredient is insoluble in the other components of the agglomerates.

The air pockets within the agglomerates may be produced from air bubbles within a molten mixture of wax and oil used to prepare the agglomerates. When the molten mixture is cooled to below its melting point, the air bubbles become trapped as air pockets with the solid agglomerates.

The air pockets comprise from 10 to 50% by volume of the agglomerates. The air pockets preferably comprise from 15%, more preferably from 20%, and most preferably from 30% by volume of the agglomerates. The air pockets preferably comprise up to 45%, more preferably up to 40%, and most preferably up to 35% by volume of the agglomerates. These amounts by volume should be understood to be averages and lead to optimum friability for the granules.

The volume of individual air pockets within an agglomerate is typically non-uniform, that is to say, there is variation in the size of the air pockets. The air pockets tend to be largely spherical in shape, although air pockets of ovoid shape are also possible. Most, that is to say at least 90% by volume, of the air pockets tend to have a maximum dimension or diameter ranging from 1 to 30% of the maximum dimension or diameter of the agglomerate in which they exist. This figure applies as an average across the agglomerates in the delivery system. Air pockets of this size tend to optimise the friability of the agglomerates.

The distribution of individual air pockets within an agglomerate is typically non-uniform, that is to say, the distribution tends to have no regular pattern.

Other components may be present in the agglomerates. A particular component that may be used is fumed silica, typically at a level of from 1 to 10% by weight of the agglomerates. This component can give manufacturing benefits.

A preferred additional component of the agglomerates is a surface coating. The coating can serve reduce leakage or seepage of the active ingredient through the surface of the agglomerates. It can also serve to hide the colour of the active ingredient, particularly when the active ingredient is a colourant.

The method of manufacture of the delivery system comprises the formation of a homogeneous mixture of oil, wax having a melting point of at least 50°C, and active ingredient. Typically, the wax is heated to temperature above its melting point and the oil and active ingredient are mixed into it. Independently, water is also heated to a temperature above the melting point of the wax.

The homogeneous mixture of wax and oil is added to water, or *vice versa,* with the incorporation of air bubbles into said homogeneous mixture, maintaining the temperature above the melting point of the wax throughout the addition.

The air bubbles may be introduced into the oil-wax mixture by any of means known in the art. A typically method involves stirring of the water at sufficient rate for it to contain air bubbles and then to add to the oil-wax mixture with continued stirring.

The resulting aqueous dispersion of agglomerates in water is then cooled to a temperature below the melting point of the wax. It is preferred that this is done quickly, in a manner that may be called crash cooling. Preferably the cooling involves a drop in temperature of more than 30°C in less than 30 minutes. Typically, the cooling is achieved by the addition of cold water (ambient temperature or less) to the dispersion.

Finally, the agglomerates are dried in order to remove at least part of water associated with them.

Surface coatings may be applied to the agglomerates using a fluid bed dryer. In such processes, the agglomerates may be blended with a coating agent (for example, titanium dioxide) and the mixture "fluidised" using hot air with a sufficient temperature to start melting the agglomerates. As the agglomerates melt, the coating agent sticks to the surface of the agglomerates and in so doing, coats them. Once the agglomerates are coated, they are left to cool and then optionally sieved to acquire the required size.

In performing coatings as described above, the quantity of coating agent needs to be greater than the quantity of agglomerates. Preferably a ratio of coating agent to agglomerate of at least 10:1 by weight is used.

The agglomerates are typically formulated into a composition. In the manufacture of said composition, it is preferred that the agglomerates are added as the final component in order to avoid premature breakage of the agglomerates.

The compositions in which the agglomerates are used typically have other components. In general, there is some form of carrier for the agglomerates. Typically, the carrier is capable of flow at the use temperature of the agglomerates, that is to say, it is a fluid. More typically it is a liquid, paste, or gel. In order for the agglomerates to survive in the carrier, it is required that the agglomerates are not soluble in the carrier.

In many compositions in which the agglomerates are used, a preferred component is a surfactant, in particular an anionic surfactant. When anionic surfactants are present in the composition, it is preferred that the agglomerates comprise carnauba wax.

In preferred embodiments, the agglomerates are used as part of an oral care compositions, in particular a toothpaste compositions.

Oral care compositions may comprise further ingredients which are common in the art. They may include:
antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.
humectants such as glycerol, sorbitol, propyleneglycol, xylitol, lactitol etc.;
binders and thickeners such as sodium carboxymethylcellulose, xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate);
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral care composition may be in any form common in the art, e.g. toothpaste, gel, mousse, aerosol, gum, lozenge, powder, cream, etc. and may also be formulated into systems for use in dual-compartment type dispensers.

### Examples

### Manufacture of Agglomerates

Agglomerates for use in accordance with the present invention were prepared in the following manner.

Keyplast Blue KG pigment (20g)(*ex* Keystone Europe Limited) was stirred into high oleate sunflower oil (48g) and the uniform dispersion added to molten carnauba wax (32g), heating further to prevent solidification of the carnauba wax.

Independently, 4L of water at 85°C was stirred with a Heidolph mixer. The mixture of the pigment, sunflower oil and canauba wax was poured into the vortex created in the stirred hot water and stirring continued for approximately 10 seconds. A dispersion of oily droplets was created in the water.

The dispersion of oily droplets was crash cooled by the addition of 1L of cold water. The resulting solidified agglomerates were dried and sieved to give the desired particle size range.

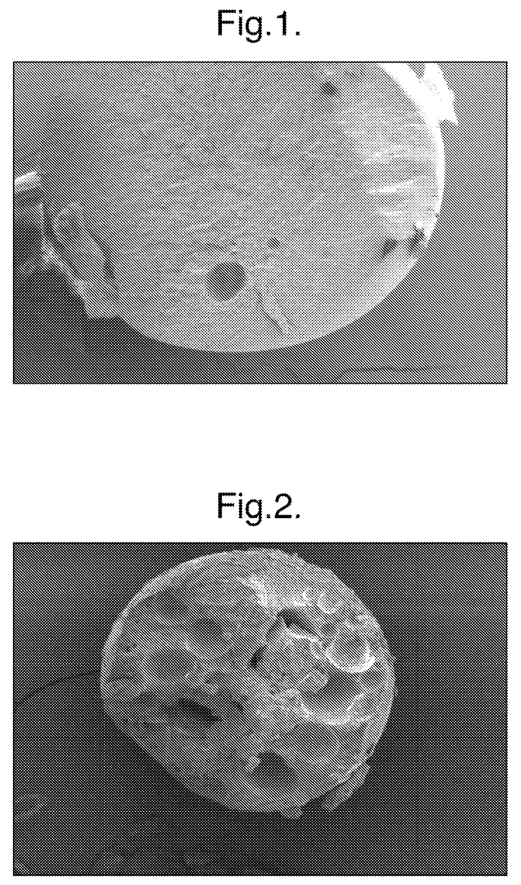

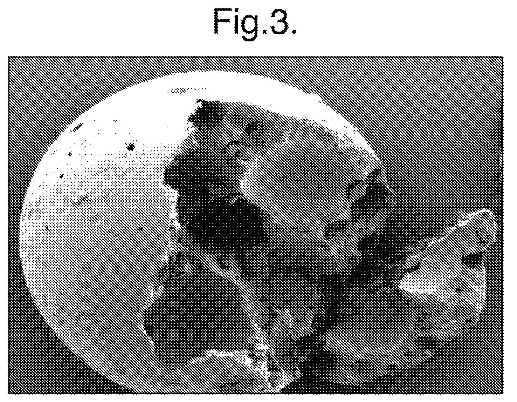

The mixture of the pigment, sunflower oil and canauba wax was poured into the hot water at a range of stirrer speeds: 250 rpm, 500 rpm, and 1000 rpm. Scanning electron microscope (SEM) images of the resulting agglomerates are shown as Figures 1, 2, and 3 respectively. It will be noted that increased stirrer speed led to increased incorporation of air pockets in the agglomerates.

### Incorporation of Agglomerates in a Toothpaste

Toothpaste compositions as indicated in Table 1 were prepared by methods known in the art. The pigment agglomerates were prepared as described above. The pigment agglomerates were the final component added to the toothpaste. They were mixed in to give a uniform dispersion.

**Table 1**

| **Component** | **Amount (% w/w)** | |
|---|---|---|
| **Composition:** | **1** | **2** |
| Sorbitol-water (70:30 w/w) | 20.0 | 20.0 |
| Saccharin | 0.3 | 0.3 |
| Flavour | 1.0 | 1.0 |
| Sodium carboxymethylcellulose | 0.02 | 0.02 |
| Preservative | 0.1 | 0.1 |
| Sodium laurylsulphate | 2.5 | 2.5 |
| Thickening silica | 3.5 | 3.5 |
| Tri-potassium citrate | 0.5 | 0.5 |
| Sodium silicate | 0.5 | 0.5 |
| Titanium dioxide | 1.0 | 1.0 |
| Chalk | 40.0 | 40.0 |
| Calcium glycerolphosphate | 0.1 | 0.1 |
| Sodium monofluorophosphate | 1.1 | 1.1 |
| Pigment agglomerates | 0.5 | 1.0 |
| **Demineralised water** | **To 100** | **To 100** |

Composition 1 contained 0.5% of the agglomerates, which in turn comprised 20% of pigment, making the amount of pigment in the total composition 0.1% by weight. Composition 2 contained twice this amount of agglomerates, equating to 0.2% by weight of pigment in the total composition.

The toothpaste compositions were placed on storage for 3 months at 45°C and monitored on a weekly basis. Excellent storage stability was observed, the toothpastes being visibly the same as when placed on storage, i.e. showing no signs of pigment leakage. In addition, the toothpastes retained the same pigment release profile when brushed onto teeth.

## Claims

1. A delivery system comprising friable agglomerates, said friable agglomerates comprising oil, wax having a melting point of at least 50°C, and active ingredient, **characterized in that** the friable agglomerates have air pockets within them, wherein the air pockets comprise from 10 to 50% by volume of the agglomerates.

2. A delivery system according to claim 1, wherein the wax has a melting point of at least 70°C.

3. A delivery system according to any of the preceding claims, wherein the oil is sunflower seed oil.

4. A delivery system according to any of the preceding claims, wherein the wax is carnauba wax.

5. A delivery system according to any of the preceding claims, wherein the agglomerates have an average yield stress of from 1 x 10⁴ to 1 x 10⁷ Pa.

6. A delivery system according to any of the preceding claims, wherein at least 90% by volume of the agglomerates have a particle size in the range of from 300 to 800 microns.

7. A delivery system according to any of the preceding claims, wherein the agglomerates comprise a surface coating.

8. A delivery system according to any of the preceding claims, wherein the agglomerates comprises from 30 to 70% by weight of oil, from 20 to 60% by weight of wax, and from 0.1 to 30% by weight of active ingredient.

9. A delivery system according to any of the preceding claims, wherein weight ratio between the wax and the oil is from 30:70 to 50:50.

10. The use of a delivery system according to any of claims 1 to 9 for the delivery of a fragrance or flavour.

11. The use of a delivery system according to any of claims 1 to 9 for the delivery of a colourant.

12. A composition comprising a delivery system according to any of claims 1 to 9 and a carrier for the agglomerates.

13. A composition according to claim 12, wherein the composition is an oral care composition and the active ingredient is a colourant.

14. The use of a composition according to claim 13, for indicating to the consumer that he has brushed his teeth for the desired time.

15. A method of delivering active ingredient comprising the incorporation of said active ingredient in friable agglomerates comprising oil and wax having a melting point of at least 50°C, the application of said friable agglomerates to the desired site of delivery, followed by the shear or pressure induced breakage of said friable granules, thereby releasing the active ingredient, **characterized in that** the friable agglomerates have air pockets within them.

16. A method of manufacture of a delivery system comprising the formation of a homogeneous mixture of oil, wax having a melting point of at least 50°C, and active ingredient, the addition of said homogeneous mixture to water with the incorporation of air bubbles into said homogeneous mixture, the homogeneous mixture and the water to which it is added both being at a temperature above the melting point of the wax, the cooling of the resulting aqueous dispersion to a temperature below the melting point of the wax, followed by the drying of the resulting waxy agglomerates.

17. A method according to claim 16, wherein the cooling step involves a drop in temperature of more than 30°C in less than 30 minutes.

18. A method according to claim 16 or claim 17, wherein the cooling step involves the addition of water at ambient temperature or less to the dispersion.

19. A method according to any of claims 16 to 18 involving the subsequent surface coating of the agglomerates using a fluid bed dryer.

20. A method according to claim 19, wherein the agglomerates are blended with a coating agent and the mixture fluidised using hot air with a sufficient temperature to start melting the agglomerates.

## Patentansprüche

1. Ein Abgabesystem, umfassend brüchige Agglomerate, wobei die brüchigen Agglomerate Öl, Wachs mit einem Schmelzpunkt von mindestens 50°C und Wirkstoff umfassen, **dadurch gekennzeichnet, dass** die brüchigen Agglomerate Lufttaschen im Inneren aufweisen, wobei die Lufttaschen 10 bis 50 Volumen-% der Agglomerate umfassen.

2. Ein Abgabesystem nach Anspruch 1, wobei das Wachs einen Schmelzpunkt von mindestens 70°C aufweist.

3. Ein Abgabesystem nach irgendeinem der vorhergehenden Ansprüche, wobei das Öl Sonnenblumenöl ist.

4. Ein Abgabesystem nach irgendeinem der vorhergehenden Ansprüche, wobei das Wachs Carnaubawachs ist.

5. Ein Abgabesystem nach irgendeinem der vorhergehenden Ansprüche, wobei die Agglomerate eine durchschnittliche Fließspannung von 1 x 10⁴ bis 1 x 10⁷ Pa aufweisen.

6. Ein Abgabesystem nach irgendeinem der vorhergehenden Ansprüche, wobei mindestens 90 Volumen-% der Agglomerate eine Partikelgröße in dem Bereich von 300 bis 800 Mikron aufweisen.

7. Ein Abgabesystem nach irgendeinem der vorhergehenden Ansprüche, wobei die Agglomerate eine Oberflächenbeschichtung aufweisen.

8. Ein Abgabesystem nach irgendeinem der vorhergehenden Ansprüche, wobei die Agglomerate 30 bis 70 Gewichts-% Öl, 20 bis 60 Gewichts-% Wachs und 0,1 bis 30 Gewichts-% eines Wirkstoffs umfassen.

9. Ein Abgabesystem nach irgendeinem der vorhergehenden Ansprüche, wobei das Gewichtsverhältnis zwischen dem Wachs und dem Öl 30:70 bis 50:50 beträgt.

10. Die Verwendung eines Abgabesystems nach irgendeinem der Ansprüche 1 bis 9 zur Abgabe eines Duftstoffs oder Aromas.

11. Die Verwendung eines Abgabesystems nach irgendeinem der Ansprüche 1 bis 9 zur Abgabe eines Färbemittels.

12. Eine Zusammensetzung, umfassend ein Abgabesystem nach irgendeinem der Ansprüche 1 bis 9 und einen Träger für die Agglomerate.

13. Eine Zusammensetzung nach Anspruch 12, wobei die Zusammensetzung eine Mundpflegezusammensetzung und der Wirkstoff ein Färbemittel ist.

14. Die Verwendung einer Zusammensetzung nach Anspruch 13, die dem Verbraucher anzeigt, dass er seine Zähne die gewünschte Zeit gebürstet hat.

15. Verfahren zur Wirkstoffabgabe, umfassend die Einverleibung des Wirkstoffs in brüchige Agglomerate, umfassend Öl und Wachs eines Schmelzpunktes von mindestens 50°C, das Aufbringen der brüchigen Agglomerate auf die gewünschte Abgabestelle, gefolgt von durch Scherung oder Druck erzeugtem Bruch der brüchigen Granalien, wodurch der Wirkstoff freigegeben wird, **dadurch gekennzeichnet, dass** die brüchigen Agglomerate im Inneren Lufttaschen aufweisen.

16. Verfahren zur Herstellung eines Abgabesystems, umfassend die Bildung einer homogenen Mischung von Öl, Wachs mit einem Schmelzpunkt von mindestens 50°C und einem Wirkstoff, die Zugabe der homogenen Mischung zu Wasser unter Einverleibung von Luftblasen in die homogene Mischung, wobei sich die homogene Mischung und das Wasser, zu dem sie gegeben ist, beide auf einer Temperatur oberhalb des Schmelzpunktes des Wachses befinden, das Kühlen der erhaltenen wässrigen Dispersion auf eine Temperatur unterhalb des Schmelzpunktes des Wachses, gefolgt von dem Trocknen der erhaltenen wachsartigen Agglomerate.

17. Verfahren nach Anspruch 16, wobei der Kühlschritt einen Temperaturabfall von mehr als 30°C in weniger als 30 Minuten einbezieht.

18. Verfahren nach Anspruch 16 oder Anspruch 17, wobei der Kühlschritt die Zugabe von Wasser bei Raumtemperatur oder weniger zu der Dispersion einbezieht.

19. Verfahren nach irgendeinem der Ansprüche 16 bis 18, das das nachfolgende Oberflächenbeschichten der Agglomerate unter Verwendung eines Fließbetttrockners einbezieht.

20. Verfahren nach Anspruch 19, wobei die Agglomerate mit einem Beschichtungsmittel gemischt und die Mischung unter Anwendung von Heißluft mit einer ausreichenden Temperatur fluidisiert wird, um das Schmelzen der Agglomerate zu starten.

## Revendications

1. Système de distribution comprenant des agglomérats friables, lesdits agglomérats friables comprenant une huile, une cire ayant un point de fusion d'au moins 50 °C, et un principe actif, **caractérisé en ce que** les agglomérats friables ont des poches d'air en leur intérieur, les poches d'air représentant de 10 à 50 % en volume des agglomérats.

2. Système de distribution selon la revendication 1, dans lequel la cire a un point de fusion d'au moins 70 °C.

3. Système de distribution selon l'une quelconque des revendications précédentes, dans lequel l'huile est une huile de graines de tournesol.

4. Système de distribution selon l'une quelconque des revendications précédentes, dans lequel la cire est la cire de carnauba.

5. Système de distribution selon l'une quelconque des revendications précédentes, dans lequel les agglomérats ont une limite apparente d'élasticité moyenne de 1 x 10⁴ à 1 x 10⁷ Pa.

6. Système de distribution selon l'une quelconque des revendications précédentes, dans lequel au moins 90 % en volume des agglomérats ont une granulométrie comprise dans la plage allant de 300 à 800 µm.

7. Système de distribution selon l'une quelconque des revendications précédentes, dans lequel les agglomérats comprennent un revêtement de surface.

8. Système de distribution selon l'une quelconque des revendications précédentes, dans lequel les agglomérats comprennent de 30 à 70 % en poids d'huile, de 20 à 60 % en poids de cire et de 0,1 à 30 % en poids de principe actif.

9. Système de distribution selon l'une quelconque des revendications précédentes, dans lequel le rapport en poids entre la cire et l'huile est de 30:70 à 50:50.

10. Utilisation d'un système de distribution selon l'une quelconque des revendications 1 à 9, pour la distribution d'un parfum ou d'un arôme.

11. Utilisation d'un système de distribution selon l'une quelconque des revendications 1 à 9, pour la distribution d'un colorant.

12. Composition comprenant un système de distribution selon l'une quelconque des revendications 1 à 9 et un support pour les agglomérats.

13. Composition selon la revendication 12, dans laquelle la composition est une composition de soin buccal et le principe actif est un colorant.

14. Utilisation d'une composition selon la revendication 13, pour indiquer au consommateur qu'il a lavé ses dents pendant la durée souhaitée.

15. Procédé de distribution d'un principe actif comprenant l'incorporation dudit principe actif dans des agglomérats friables comprenant une huile et une cire ayant un point de fusion d'au moins 50 °C, l'application desdits agglomérats friables sur le site souhaité de distribution, suivie par une rupture induite par cisaillement ou pression desdits granulés friables, libérant ainsi le principe actif, **caractérisé en ce que** les agglomérats friables ont des poches d'air en leur intérieur.

16. Procédé de fabrication d'un système de distribution comprenant la formation d'un mélange homogène d'huile, de cire ayant un point de fusion d'au moins 50 °C et d'un principe actif, l'ajout dudit mélange homogène à de l'eau avec l'incorporation de bulles d'air dans ledit mélange homogène, le mélange homogène et l'eau à laquelle il est ajouté étant tous deux à une température supérieure au point de fusion de la cire, le refroidissement de la dispersion aqueuse résultante à une température inférieure au point de fusion de la cire, suivi par le séchage des agglomérats de cire résultants.

17. Procédé selon la revendication 16, dans lequel l'étape de refroidissement implique une chute de température supérieure à 30 °C en moins de 30 minutes.

18. Procédé selon la revendication 16 ou la revendication 17, dans lequel l'étape de refroidissement implique l'ajout d'eau à la dispersion à température ambiante ou à une température inférieure.

19. Procédé selon l'une quelconque des revendications 16 à 18, impliquant le revêtement de surface ultérieur des agglomérats en utilisant un séchoir à lit fluidisé.

20. Procédé selon la revendication 19, dans lequel les agglomérats sont mélangés avec un agent de revêtement et le mélange fluidisé en utilisant de l'air chaud avec une température suffisante pour initier la fusion des agglomérats.
